# EUROPEAN PATENT SPECIFICATION

(11) **EP 1 267 826 B1**
(45) Date of publication and mention of the grant of the patent: **03.01.2007**
(21) Application number: 00968217.0
(22) Date of filing: 22.10.2000
(51) Int. Cl.: A61K 9/00, A61K 47/00, A61K 47/30, A61K 31/65, A61P 31/04

(54) **PHARMACEUTICAL COMPOSITION FOR TREATMENT OF MUCOSAL EPITHELIAL ULCERATION AND/OR EROSION**
PHARMAZEUTISCHE ZUSAMMENSETZUNG ZUR BEHANDLUNG VON ULZERATIONEN UND/ODER EROSIONEN DES SCHLEIMHAUTEPITHELS
COMPOSITION PHARMACEUTIQUE POUR LE TRAITEMENT DE L'ULCERATION ET/OU EROSION EPITHELIALE DES MUQUEUSES

(30) Priority: 22.10.1999 IS 522899
(43) Date of publication of application: 02.01.2003
(73) Proprietor: LIF-HLAUP EHF. BIO-GELS PHARMACEUTICALS INC., 107 Reykjavik (IS)
(72) Inventor: SKULASON, Skuli, IS-112 Reykjavik (IS); HOLBROOK, W., Peter, IS-105 Reykjavik (IS); KRISTMUNDSDOTTIR, Thordis, IS-170 Seltjarnarnes (IS); GIZURARSON, Sveinbjorn, IS-107 Reykjavik (IS)
(74) Representative: Olsen, Lars Pallisgaard
(86) International application number: PCT/IS2000/000013
(87) International publication number: WO 2001/028515

(56) References cited:
- EP-A2- 0 125 759
- EP-A2- 0 288 945
- WO-A1-93/21905
- WO-A1-96/09829
- WO-A1-98/17251
- WO-A2-99/45910
- DE-A1- 4 434 929
- DE-A1- 19 536 411
- GB-A- 1 216 268
- US-A- 4 894 232
- DATABASE CAPLUS [Online] DOC. NO. 84:95585 KORBAR-SMID JELKA ET AL.: 'Oxytetracycline formulation to be applied at the oral mucosa', XP002956069 Retrieved from STN International Database accession no. 1976:95585 & ACTA PHARM. JUGOSL. vol. 25, no. 4, 1975, pages 271 - 276
- Reynolds J.E.F. Martindale, The extra Pharmacopoeia, 30th eidition, The Pharmaceutical Press, Lonodn 1993, page 215

## Description

### 1. BACKGROUND OF THE INVENTION

The present invention relates to a bioadhesive pharmaceutical composition for treatment of mucosal epithelial ulceration and/or erosions; use of the composition for the manufacture of a medicament for treating mucosal ulceration and/or erosion; a method of treating mucosal epithelial ulceration and/or erosion in a mammal; and a method of delivering a biologically active substance to the mucosa of a mammal for treatment of mucosal epithelial ulceration and/or erosion.

### The Technical Field

### Buccal Ulcerations

The Dental Practitioners/ British National Formulary (DPF 1982-4/BNF No. 4 1982) recommends a bacteriostatic concentration of tetracycline (125mg/5ml) as a mouth bath for the treatment of mucosal ulceration. This extremely high concentration of 25mg/ml (2.5% w/v) and the poor bioflux of the formulation will result in a considerable waste of drug, cause a high risk of affecting the microbiological balance of the mucosa and may induce bacteriological resistance to this drug. Also, the formulation does not have bioadhesive properties and, therefore requires high concentration of tetracycline in order to achieve passive influx of the drug in sufficient amounts through the mucosa and into the epithelium.

Other therapeutic methods are based on the use of doxycycline, Periostat^{®} or the injectable doxycycline, Atridox^{®}. These formulations are designed for peroral/gingival sulcal administration and not for topical delivery to the mucosa.

Topical administration to the oral mucosa of formulations containing tetracycline or tetracycline-like substances as the active ingredient have been designed to treat periodontal disease or infectious conditions by delivering bacteriostatic concentrations of the antibiotic in the gingival crevice with slow release of the active ingredient over the treatment period. E.g. EP 0 125 759 uses 1% (w/w) of tetracycline base, a fixative agent, e.g. 10% (w/w) iota-carregeenan, and excipients, e.g. 1,5% (w/w) hydroxyethyl cellulose and 0,7%(w/w) buffer salts, and the rest water (Example 6) for antifungal activity. With the intention of having antifungal activity as opposed to the present invention. This also differs from present invention in the amount of active ingredient being 1% opposed to that of the present invention which is selected to ensure that the normal flora is not affected.
Also, in order to exploit anti-inflammatory properties, although not mentioned as action intended by producers, rather than bacteriostatic properties of doxycycline and doxycycline-like substances, formulations (e.g. Atridox^{®}) have been used to release sub-inhibitory doses of doxycycline (100mg/g) (10.0% w/w) to the gingival crevice for the treatment of periodontal disease.

In the treatment of various periodontal diseases, including mucosal diseases, tetracyclines have been used as adjuncts the doses (≥125mg/dose) of which have affected the natural oral bacterial flora (normal flora). Although they produce a considerable relief of symptoms, the long-term therapy and high dosage induce serious side effects, such as a high incidence of bacterial resistance to the drug, and frequent fungal infections due to suppression of natural bacterial flora (Kornman et al., *Journal of Periodontology,* 53, 604-610, 1982). A number of terms have been used to describe the characteristic mixtures of microorganisms associated with a specific site, these include, normal, indigenous, natural, resident or commensal flora (Marsh P, Oral Microbiology 4^{th} ed., Reed Educational and Professional Publishing Ltd. 1999), here after in this text the word normal flora will be used when referring to this subject.

In EP 0 184 389 there is disclosed a stable composition containing minocycline for treating periodontal diseases, said composition comprising a magnesium compound as stabiliser. A method of treating periodontal diseases includes injection thereof into the inside of periodontal pockets. Nothing is indicated or suggested about treating the mucosal epithelial ulceration and/or erosions, nor adaptation of the release of biologically active substance so as not to. affect the normal flora of the mucosa.

Another invention DE 4 434 929 describes the use of nystatine (2-3%) to treat periodontal diseases using nystatine as the BAS and carbopol 934 as an excipient. As opposed to tetracycline nystatine does not have an MMP inhibitory effect. And the effort is focused on the bactericidal effect as the mechanism they are looking for, where the current invention focuses on the MMP inhibitory effect - a significantly different mechanism and in significantly lower doses of BAS (0,1-0,3%). The excipient carbopol 934 is mentioned and used in both inventions is another similarity. In present invention, however, carbopol is used in 0,5-1,5% concentration, where the German invention teaches that it should be used in 25-30% concentration.

GB-A-1 216 268 discloses an adhesive composition for mucous membranes and cutaneous surfaces, the composition comprising an anhydrous composition of Carbopol 934 in an anhydrous environment and an antibiotic from the tetracycline series in combination with a steroid which; such a steroid promoting fungal infection and consequently affects the normal flora.

STN "CAPLUS", accession number 1976:95, 585, teaches a composition comprising 2,5 by weight of oxytetracycline and gelatin for the treatment of stomatitis and gingivitis, i.e. inflammatory diseases of the oral mucosa, and especially of gingival, e.g. caused by bacteria or fungi.

WO 99/45 910 teaches MMP inhibitor formulations to be administered topically in dosages (lower limits) of 0.1% to 5% for the treatment of mucositis, the formulations including a combination of the MMP inhibitor and an anti-inflammatory agent and/or an antimicrobial agent whereby the oral microbial flora intentionally will be affected.

### Matrix Metalloproteinases (MMP'S)

MMP's are enzymes that take part in many inflammatory processes in the human body and promote breakdown of tissues and fibers when their natural inhibitors do not correctly regulate them. Over ten years ago scientists discovered that the tetracyclines not only possess antibacterial properties but also inhibit MMP's (Golub et. al., *Journal of Periodontal Research,* 18, 516-526, 1983).

Examples of pathological conditions characterized by excessive MMP activity include: osteoporosis; various arthritis, including rheumatoid, osteo- and reactive arthritis; bullous and ulcerative skin diseases including recessive dystrophic epidermolysis bullosa; α₁-antitrypsin-deficiency paniculitis; dermatitis herpetiformis; and pyoderma gangrenosum; sterile corneal ulcers; various complications of diabetes mellitus; and tumor-induced angiogenesis; invasion and metastasis as described in recent review publication by Ingman et al. (Ingman et al., *Journal of Periodontology,* 64, 82-88, 1993). Other physiological events that MMP's are involved in are such as embryologic development, tissue remodelling, salivary gland morphogenesis, and tooth eruption (Ryan et al., *Current Opinion in Periodontology,* 3, 85-96, 1996).

The structure of the MMP's is generally divided in five parts: the signal peptide, propeptide, catalytic site, hinge region, and pexin like domain. When activated the enzyme first loses the signal peptide and then the internal bonds of the propeptide are disrupted. The disruption of the Cystein-Zn⁺⁺ bond is a prerequisite to the activation of the enzymes, which allows Zn⁺⁺ to catalyze one or more cleavages (Birkedal-Hansen, *Journal of Periodontology,* 64, 474-484, 1993).

It has been found that of the commercially available tetracyclines, doxycycline has shown the best inhibition of the MMP's (Burns et al., *Investigative Ophthalmology & Visual Science,* 30, 1569-1575, 1989). The mechanism of this inhibition was first proposed to be the ability of the tetracyclines to inhibit already active MMP's, which was shown by Golub et al. (Golub et al., *Journal of Periodontal Research,* 18, 516-526, 1983). Later it was found that the tetracyclines also inhibited pro-MMP activation and also down-regulated the expression of MMP's (Ryan et al., *Advances in Dental Research* 12: 149-151, 1998). Chemically modified tetracyclines (CMT) have been made to rid the tetracyclines of the anti-bacterial properties and to identify the site of the anticollagenase property (Golub et al., *Critical Reviews in Oral Biology & Medicine,* 2, 297-322, 1991). This study revealed that CMT-5, a pyrrazole analogue, did not have any anticollagenase properties. Here the carbon group in position 11 and hydroxyl group in position 12 had been removed, thus it appeared that the carbonyl and the hydroxyl groups at C-11 and C-12, respectively, might be essential for the anticollagenase property of these drugs. Other chelating chemicals have shown inhibition of MMP's, like EDTA and TMB-8 (Gabler et al., *Journal of Periodontal Research,* 26, 52-58, 1991).

### Tetracycline MMP Inhibitors

Studies using tetracyclines in formulations indicate that tetracyclines may act as MMP inhibitors. Thus, Ciancio used low-dose doxycycline therapy in patients with adult periodontitis (Ciancio, *Advances in Dental Research,* 12, 27-31, 1998). Other studies, such as wound healing after topical application of CMT-2 (Ramamurthy et al., *Advances in Dental Research,* 12, 144-148, 1998), bone resorption in rats (Chang et al., *Journal of Periodontal Research,* 29, 242-249, 1994), suppression of human neutrophil functions (Gabler et al., *Journal of Periodontal Research,* 26, 52-58, 1991), mouse renal adenocarcinoma and metastasis (Masumori et al., *The Journal of Urology,* 151, 1400-1404, 1994), inhibition of collagenolytic activity of extracts from human osteoarthritic cartilage (Yu et al., *The Journal of Rheumatology,* 18, 1450-1452, 1991), and the effect of tetracyclines on collagenases activity in patients with recurrent aphthous ulcers (Häyrinen-Immonen et al., *Journal of Oral Pathology & Medicine,* 23, 269-272, 1994), also show the MMP activity of tetracyclines. It has also been shown that in low doses the tetracyclines do not induce the emergence of tetracycline resistance bacterial strains (Schroeder et al., *Journal of Dental Research,* 71, 758(1936), 1992; Thomas et al., *Journal of Dental Research,* 74, 575(1394), 1995).

### 2. DISCLOSURE OF THE INVENTION

It is the object of the present invention to provide a pharmaceutical composition and method for treatment of mucosal epithelial ulceration and/or erosions without affecting the normal flora of the mucosa.
It is another object of the present invention to provide such a pharmaceutical composition and method for treatment of mucosal epithelial ulceration and/or erosions which reduces amount of biologically active substance and which reduces the risk of side effects.

Further objects of the present invention appear from the disclosure elsewhere in the description.

According to the invention, this object is fulfilled by providing a bioadhesive pharmaceutical composition for treatment of mucosal epithelial ulceration and/or erosions as claimed in claim 1; said hydrogel composition comprising:
(i) a bioadhesive substance;
(ii) a biologically active substance having MMP inhibitory activity in the range 0.05 to 0.5% (w/w) of the composition; and
(iii) an optional physiologically acceptable vehicle;
said bioadhesive substance and optional physiologically acceptable vehicle being selected to release the biologically active substance in a therapeutic amount into the epithelia of the mucosa without affecting the normal flora thereof. Within the present invention the term "without affecting the normal flora" is intended to mean, that the normal flora of the mucosa being treated will be the same prior to and after treatment with the present invention. This also indicates that a fungal infection will not occur as a result of an unbalanced normal flora.

It is predictable that inhibition of bacterial growth will occur when in direct contact with bacteria, given the nature of the active substance, when being tetracycline or any of it's derivatives, this is a positive action in the core of ulceration and as shown in example 5 it does not have this marked effect on the bacteria when in the mixed normal flora nor following diffusion from the base of the ulcer or other lesions.

This object is further solved by providing use of the pharmaceutical composition according to the invention for the manufacture of a medicament for treatment of mucosal epithelial ulceration and/or erosion of humans.
Generally, the administration of the biologically active substance can be at any suitable location of the mucosa. In a preferred embodiment, the administration is to a surface of a mucosa whereby a site-specific action is obtained and systemic action is prevented.

Also, this object is further solved by providing the use of a composition according to the invention for treating mucosal epithelial ulceration and/or erosion in a mammal ; or to provide the use of a composition according to the invention for delivering a biologically active substance to the mucosa of a mammal for such a treatment.

### "Mucosal Epithelial Ulceration and/or Erosions"

The pharmaceutical formulation is intended for use as a treatment of numerous inflammatory and/or ulcerative conditions of the oral mucosa, both chronic and acute. These disorders have widely differing aetiologies but have in common the destruction of mucosa in which MMP's are thought to play a part, as for example in aphthous ulcerations where it has been shown that MMP-8 is the predominant collagenase at the core of the ulcers (Häyrinen-Immonen et al., *Journal of Oral Pathology & Medicine,* 23, 269-272, 1994). The invention is not intended for treating deep ulcers extending beyond the lamina propria into muscle, fat, bone or other underlying tissues. The pharmaceutical formulation is applied, preferably as a bioadhesive hydrogel composition, to the affected area of the mucosa which may be moist or predried. Increased adhesion makes the treatment more effective. The action of the bioadhesive composition is the inhibition of MMP's that in turn prevents further destruction of tissue that is attempting to repair.

Diseases in mammals and humans that are amenable to therapy with the bioadhesive pharmaceutical composition according to the invention, preferably bioadhesive hydrogel composition, include primary herpes simplex (herpetic gingivo-stomatitis); secondary (recurrent) herpes e.g. in the oral cavity, not least where this condition appears in immuno-compromised patients; major and minor aphthous ulceration; herpetiform ulceration herpangina hand, foot and mouth disease and other manifestations of coxsackie virus infections in the oral cavity or other mucosal surfaces; mucosal manifestation of erythema multiforme and variations of this condition in the oral cavity or other mucosal surfaces; lichen planus including erosive (ulcerative) and atrophic forms of oral lichen planus as well as lichen planus conditions on other mucosal surfaces; other forms of intra-mucosal ulceration including mucous membrane pemphigoid.

According to the invention there is provided a pharmaceutical composition for local delivery of a biologically active substance (BAS) for the treatment of mucosal epithelial ulceration and/or erosions.

These conditions include various inflammatory and ulcerative diseases of mucous membranes, particularly the oral mucous membrane. The diseases include minor aphthous ulceration; major aphthous ulceration; primary herpes infection; coxsackie viral infections; erythema multiforme; mucous membrane pemphigoid and erosive/ atrophic forms of lichen planus.

Generally, the diseases include that the mucosa is inflamed, ulcerated or that it has surface erosion. In a preferred embodiment, the ulcers and erosions extend through part of or the entire surface epithelial layer of the mucosa but only just into the underlying lamina propria.

The pharmaceutical composition according to the invention is not intended for treating deep ulcers extending beyond the lamina propria into muscle, fat, bone or other underlying tissues.

Further, it is not intended for the treatment of periodontal disease with the exception of (i) acute gingivitis caused by herpes virus infection; (ii) other viral infections of the gingiva; (iii) desquamative types of gingivitis such as in oral manifestations of pemphigoid and lichen planus.

Generally, the pharmaceutical composition according to the invention can be applied to any suitable mucosa. In a preferred embodiment, the mucosal surface is selected from the group of mucosal surfaces of the nose, lungs, mouth, eye, ear, gastrointestinal tract, genital tract, vagina and rectum.

### "Biologically Active Substance"

The biological active substance is a substance having matrix metalloproteinase (MMP) inhibitor activity.

The proposed biologically active substance may be a tetracycline or its derivative in a concentration below antibacterial use. The BAS may also be an enzyme inhibitor such as MMP inhibitor.

Preferably, biologically active substance is a low-dose tetracycline or any of its derivatives, which have virtually no antimicrobial activity and is therefore less likely to induce the emergence of resistant strains of bacteria.

Preferred tetracyclines may be selected from, but not limited to tetracycline, doxycycline, minocycline, oxytetracycline, chlortetracycline, methacycline, demeclocycline or any other chemically modified tetracycline.

In a preferred embodiment, the biologically active substance is selected from the group consisting of tetracycline's or its derivatives such as tetracycline, doxycycline, minocycline or oxytetracycline, and in a concentration below antimicrobial concentration.

The combination of bioadhesive properties of the bioadhesive substance and release time of the biological active substance is adapted to induce a therapeutic effect.

It is preferred that the biologically active substance is released in a therapeutic amount into the epithelia of the mucosa without affecting the normal bacterial flora.

The concentration of the biologically active substance is selected to provide the therapeutic effect without affecting the normal flora.

The concentration of the biologically active substance is in the range 0.05 to 0.5%, preferably 0.1 to 0.5%, most preferred 0.1 to 0.3% (w/w) of the composition.

Generally the biologically active substance can be in any suitable form.

In a preferred embodiment, the biologically active substance is in a dissolved form whereby it is more stable than in a particulate form.

In another preferred embodiment, the biologically active substance is in a particulate form whereby it is more stable than in a dissolved form.

### "Additional Active Ingredients"

In addition to releasing the biologically active substance having MMP inhibitory activity, the composition may comprise one or more additional active ingredients.

In a preferred embodiment, the one or more additional active ingredients is selected from the group consisting of anesthetics including benzocaine, pramoxine, dibucaine, diclonine, lidocaine, mepiracaine, prilocaine and tetracaine; astringents including calamine, zinc oxide, tannic acid, hamamelis water, zinc sulfate; wound cleansers such as benzalkonium chloride, carbamide peroxide, tannic acid, salicylic acid, triclosan, benzoyl peroxide and boric acid; wound healing agents such as fish oils, shark liver oil, castor oil, sucralfate and liver yeast cell derivatives; antihistamines such as diphenhydramine, promethazine, cromolyn, cyproheptadine, and azatadine.

### "Bioadhesive Substance"

The combination of bioadhesive properties of the bioadhesive substance and release time of the biological active substance is adapted to induce a therapeutic effect.

Generally, the concentration of the bioadhesive substance is selected to provide a specific release profile of the biologically active substance to achieve the therapeutic effect.

In a preferred embodiment, the concentration of the bioadhesive substance is in the range 0.05 to 10%, preferably 0.1 to 8%, most preferred 0.1 to 5% (w/w) of the composition.

Generally, the bioadhesive substance can be any substance with bioadhesive properties to mucosal membranes.

The pharmaceutical composition can comprise one or more bioadhesive components.

In a preferred embodiment, the one or more bioadhesive components are selected from the group consisting of polysaccharides, such as hydroxymethylcellulose, hydroxypropylmethylcellulose, hydroxyethylcellulose, carboxymethylcellulose, and salts thereof; acrylic polymers such as polyacrylic acids, polymethacrylates, poly(hydroxyethyl methacrylate), poly(methoxyethyl methacrylate), poly(methoxyethoxyethyl methacrylate); proteins such as gelatin; high molecular weight polyhydroxy compounds such as polyvinyl alcohols; high average molecular weight polyalkylene glycols such as polyethylene glycols, optionally cross-linked in particular being with an average molecular weight from about 20,000 to about 4,000,000, propylene glycole, optionally cross-linked with hydrophilic or hydrophobic groups such as glycofurolum; polyvinylpyrrolidones, in particular having an average molecular weight in the range from 10,000 to about 700,000; chitosans, pectins, xanthan gums, carragenans, locust bean gums, acacia gums, and alginates.

In a particularly preferred embodiment, the bioadhesive substance comprises one or more bioadhesive components selected from the group of bioadhesives/water gelling agents such as Carbopol 934, Carbopol 940, Carbopol 941, Carbopol 971, Carbopol 974, Carbopol 980, Carbopol 981, Carbopol 1342, Carbopol 1382, carboxymethylcellulose and hydroxypropylmethylcellulose and salts thereof, xanthan gums, polycarbophil and mixtures thereof.

Pharmaceutically acceptable excipients may include emulsifying agents, surfactants, antioxidants, enzyme inhibitors, buffering agents, preservatives, humectants, chelating agents, gel forming agents, organic solvents, oils, viscosity controlling agents, HLB-controlling agents, ointment bases, osmotic pressure controlling agents, propellants, air displacement, water and mixture thereof. The BAS can optionally be administered in a pharmaceutically or physiologically acceptable vehicle, such as physiological- or phosphate buffered saline, water, dextrose, ethanol polyols (such as glycerol or propylene glycol), and combinations thereof.

Examples of antioxidants are butylated hydroxy anisole (BHA), ascorbic acid and derivatives thereof, tocopherol and derivatives thereof, and cysteine. Examples of preservatives are parabens, such as methyl or propyl p-hydroxybenzoate, chlorobutanol, benzyl alcohol, phenol, cetylpyridinum chloride and benzalkonium chloride. Examples of substances, which inhibit enzymatic degradation, are aprotinin, DFP. Examples of humectants are glycerine, propylene glycol, sorbitol, and urea.

Examples of chelating agents are sodium EDTA, citric acid and phosphoric acid. Furthermore pH-controlling agents may be selected from acetic acid, hydrochloric acid, nitric acid, potassium metaphosphate, potassium phosphate, sodium acetate, ammonia, sodium carbonate, sodium hydroxide, sodium borate, and trolamine. Osmotic pressure controlling agents may be selected from dextrose, sodium chloride, mannitol; and propellants may be selected from dichlorodifluoromethane, dichlorotetrafluoroethane, trichloromonofluoromethane and other non-ozone damaging propellants such as butane. Air displacement may be nitrogen. Substances, which inhibit enzymatic degradation, may be selected form aprotinin, DFP. Solubilizers may be selected from alcohol, isopropyl alcohol, water, glycofurol, low molecular weight polyethylene glycols and stabilizers such as cyclodextrines. Examples of HLB-controlling agents are Tween 20-85, Span 20-80, Brij 30-98 and acacia. Examples of other excipients are oils including almond oil, castor oil, cacao butter, coconut oil, corn oil, cottonseed oil, linseed oil, olive oil, palm oil, peanut oil, poppy seed oil, rapeseed oil, sesame oil, soybean oil, sunflower oil, teaseed oil and Myglyols. Examples of ointment bases are beeswax, paraffin, cetyl palmitate, vegetable oils, and polyethylene glycols. Formulations of the invention are suitable for direct application or for introduction into relevant orifice(s) of the body, e.g. the rectal, urethral, vaginal or oral orifices.

The hydrogel preparation according to the invention can provide the absorption promoting agent in admixture with a dispersing or wetting agent, suspending agent, and/or one or more preservatives.

Suitable dispersing or wetting agents are, for example, naturally occurring phosphatides, e.g., lecithin, or soybean lecithin; condensation products of ethylene oxide with e.g. a fatty acid, a long chain aliphatic alcohol, or a partial ester derived from fatty acids and a hexitol or a hexitol anhydride, for example polyoxyethylene stearate, polyoxyethylene sorbitol monooleate, polyoxyethylene sorbitan monooleate etc. Suitable suspending agents are: naturally occurring gums such as, e.g., gum acacia, xanthan gum, or gum tragacanth; celluloses such as, sodium carboxymethylcellulose, microcrystalline cellulose (e.g. Avicel RC 591), methylcellulose; alginates such as sodium alginate, and others.

For application to the mucosa, the formulations according to the invention may contain conventional, non-toxic, pharmaceutically acceptable carriers and recipients including microspheres and liposomes. The formulations include hydrogels. In one embodiment, the formulation can be administered as nasal spray, nasal drops, nasal powder, nasal foam or as nasal ointment to the mucosal surface or the adenoids of the nose or as oral spray, oral drops, oral powder, oral patch, oral film, oral foam or as oral ointment especially directed to the buccal area. The formulation can also be in the form of eye drops or eardrops.

For application to the nasal mucosa, nasal sprays and aerosols for inhalation are suitable compositions for use according to the invention. In a typical nasal formulation, the biologically active substance is present in the form of a solution or particulate formulation in a suitable vehicle.

The pharmaceutically acceptable vehicles and excipients and optional other pharmaceutically acceptable materials present in the composition such as diluents, flavoring agents, preservatives and the like are all selected in accordance with conventional pharmaceutical practice in a manner understood by the persons skilled in the art. After administration of a nasal formulation according to the invention, the biologically active substance may be absorbed through the nasal mucosa.

Other mucosal surfaces, which are suitable for the administration of formulations of the invention, are the nose, lungs, eye, ear, gastrointestinal tract, genital tract, vaginal or rectal. For application to the rectal or vaginal mucosa, suitable formulations for use according to the invention are hydrogels ; various additives can also be incorporated.

### "Release Profile"

The pharmaceutical composition according to the invention provide a relatively rapid release of the BAS from the carrier, to the area where the disease to be treated is located. This will achieve the aim of obtaining therapeutic doses of the BAS at the required site promptly and with less salivary dilution than would occur with aqueous mouth wash preparations.

As the diseases to be treated are acute, semi-acute or are acute exacerbations of chronic diseases, a rapid therapeutic dose can be administered over a period of a few days to a few weeks.

In a preferred embodiment, the bioadhesive substance and the optionally physiologically acceptable vehicle are adapted to release about 10% of BAS into the tissues within at least 10 minutes, preferably release about 20% of the BAS within 30 minutes thereby achieving a release which is much longer than that for prior art aqueous mouth bath.

### "Form of the Pharmaceutical Composition"

The suitable formulation for use according to the invention is a hydrogel formulation. A detailed discussion of hydrogels for vaginal and oral application is found in Knuth et al. (Knuth et al., Hydrogel delivery systems for vaginal and oral applications. *Advanced Drug Delivery Rewievs,* 11, 137-167, 1993). Hydrogels are established using hydrophilic natural or synthetic polymers that have the ability to swell in aqueous environment without any substantial dissolution. The preferred hydrogels are compositions with good bioadhesive properties to the mucous membranes of the oral cavity.

The presently preferred formulation compromises a gel or gel-like composition, in particular a hydrogel, which will be discussed in greater detail below, but it is anticipated that formulations which can be applied to and remain in contact with mucosa, may also be useful. Examples of other formulations are liquids or pastes of sufficient viscosity, typically a dynamic viscosity above 30.0 cP(centipoises) at 25°C, based on such liquids, pastes or foam formulations, including expanding foam formulations, and often delivered from an aerosol product. It is believed to be an important feature of any such composition that the composition is capable of being applied to and remain for a relevant period of time on the mucosa being treated.

In a preferred embodiment, the composition consists of a pharmaceutical formulation containing a hydrogel or a gel with bioadhesive properties to mucosal membranes whereby it releases the BAS to the site of application and systemic action is prevented.

### "Application of the Pharmaceutical Composition"

Application of the active component is topical. Therapy may be for short, intermediate or long periods either in continuous or intermittent treatment schedules depending on the nature of the disease being treated. The formulation, which is prepared in accordance with the present invention, comprises the use of the following main components: a substance having bioadhesive properties; and a biologically active substance having MMP inhibitory activity; and an optional physiologically acceptable vehicle.

The formulation can be administered to mucosal surfaces in humans or animals by a variety of routes including, but not limited to, by sublingual, oral, vaginal and rectal routes. In a preferred embodiment, the composition is administered to vaginal, buccal or rectal mucous membrane. The amount of biologically active agent employed in such formulation will vary depending upon the identity of the biologically active substance.
The method of the present invention comprises administering to a mammal, particularly a human, pets or breeding animals, a pharmacologically effective dose of a biologically active substance and a pharmaceutical formulation according to the invention. For example, doxycycline doses of from about 0,5mg/g to about 5mg/g, and more particularly from about 0,5mg/g to about 1,5mg/g will typically be effective to provide an MMP inhibitory effects; however, variations in these dosage ranges will occur depending upon the particular formulations and biologically active agent. Moreover, the particular dosage will depend upon the age and medical condition of the mammal to be treated, as well as on the method of administration. The skilled researcher will readily determine suitable doses.

For liquid compositions it is essential that the effective amount of the biologically active substance can be administered in an appropriate volume. For buccal administration the volume should not exceed about 1000µl for a human subjects. A larger volume can be disagreable to the patient and will drain out to the pharynx and swallowed. The result is that a part of the biologically active substance is lost from the effective site. The volume is preferably from about 10µl to about 500 µl. For the administration to the eye, nose and the ear a volume not exceeding 300 µl should be used. For the administration to the lungs, a volume not exceeding 2 ml should be used. For the administration to the vagina, a volume not exceeding 30 ml should be used. For administration to the gastrointestinal tract a volume not exceeding 100 ml should be used.

The formulation according to the invention is especially suitable for humans, including infants (except with tetracycline's as BAS), children(except with tetracycline's as BAS), adolescents, teenagers, adults and elderly. The nature of the formulation provides the ability to enhance the absorption of a variety of biologically active agents, and therefore the formulation may be used for subjects with various conditions such as humans with disease, e.g., splenectomized subjects, subjects with cancer, subjects using anticancer drugs, subjects using antibiotics, subjects using anti-inflammatory drugs, subjects with hyper-and hypothyroidism, subjects having problems with malabsorption such as diarrhea.

The formulation according to the invention is also suitable for administration to animals such as horses, sheep, dogs, cats, cows, pigs, goats, rabbits, wild animals and laboratory animals such as mice, rats, guinea pigs, hamsters, rabbits, dogs, cats or monkeys; For animals, the concentration of each component may need to be adjusted. For example for dog, the buccal cavity has extremely high humidity, which may require addition of water absorbing excipients to the formulation.

### 3. BRIEF DESCRTIPTION OF THE DRAWINGS

In the following, by way of examples only, the invention is further disclosed with detailed description of preferred embodiments. Reference is made to the drawings in which
FIG.1 illustrates MMP inhibition activity of doxycycline at various concentrations (example 1).
FIG.2 illustrates a diffusion profile (%) of doxycycline from formulation A through a synthetic membrane versus time (example 8).
FIG.3 shows a release profile (%) of doxycycline from formulation A versus time (example 7).

### 4 DETAILED DESCRIPTION OF THE INVENTION

### "Formulation of the Pharmaceutical Composition"

The biologically active agent such as the MMP inhibitory substance may be used in a particulate form or dissolved. The formulation is especially suitable for dissolved BAS; however, particulate forms are also easy to prepare into the formulation.

The effect on the natural bacterial flora of the BAS according to the invention, with a particular biologically active agent, can be assessed using methods known in the art, such as described in Example 4.

Typically the BAS will be administered with a formulation, either in the same admixture or composition, or at the same time but in a separate composition or formulation.

Comparisons of the use of BAS in the formulation according to the invention and other methods can be performed by routine methods, such as evaluating the bacterial flora prior to and after the treatment using the bacteriological method described in Example 4.

The following Examples are offered for the purpose of illustrating the present invention and are not to be construed to limit the scope of this invention.

### Example 1 (MMP INHIBITION)

In checking the inhibition of the doxycycline on MMP's a Zymography method was used (Mackay et al., *Cancer Res.,* 50(18), 5997-6001, 1990). The concentration of doxycycline needed to fully inhibit the gelatinolytic activity of MMP-2 and MMP-9 using this method.

Cell culture: HT-1080 is human fibrosarcoma cell line which is known to secrete a large amount of several matrix-degrading enzymes, among which are MMP-2 and MMP-9 (Kawamata et al., *International Journal of Oncology,* 13, 699-704, 1998). The MMP inhibition of doxycycline was tested on MMP-2 and MMP-9, which were acquired from HT-1080, and showed that complete inhibition was achieved at 50µM (24µg/mL) for MMP-9 and at 100µM (48µg/mL) for MMP-2.

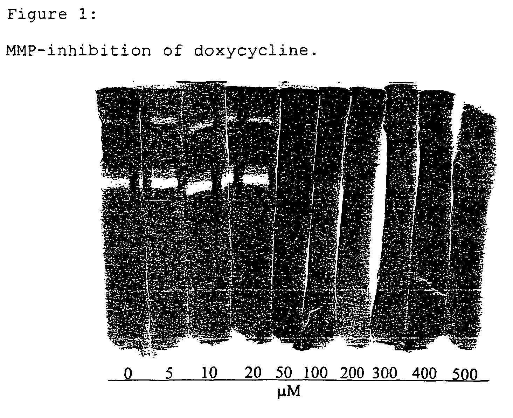

As seen in figure 1, the gels were exposed to different concentration of doxycycline solution and after development, the result were determined visually.

### Example 2 (Formulation)

The compositions according to the invention were manufactured according to table below:

| Component: | Formulation A | Formulation B |
|---|---|---|
| Carbopol 934 | X (1,0%) | |
| HPMC | X (0,48%) | |
| Chlorhexidine 20% | | X (4,44%) |
| Xanthan Gum | | X (3,5%) |
| Doxycycline, HCl | X (0,15%) | X (0,15%) |
| Propylene glycol | X (10%) | X (10%) |
| Methylparahydroxybenzoat | X (0,08%) | X (0,08%) |
| Propylparahydroxybenzoat | X (0,02%) | X (0,02%) |
| EDTA | X (0,1%) | X (0,1%) |
| Natrii Pyrosulfis | X (0,15%) | X (0,15%) |
| Distilled water | X (ad 100%) | X (ad 100%) |

Bioadhesives and gelling agents are dissolved in warm water and stirred overnight while the solution cools; the preservatives are dissolved in propylene glycol. These gel solutions are combined under cautious stirring and when a homogenous solution is achieved the propylene glycol solution is added. The pH of the composition is adjusted to around pH 7 with NaOH. Antioxidants along with the active ingredient are dissolved in the water, which is needed to achieve the correct weight. The hydrogel is then centrifuged to remove air bubbles.

The hydrogel is preferably stored in an airtight (hermetic) and light-resistant container, to preserve the drug from oxygen and light that might induce oxidation of the active ingredient.

### Example 3 (Bacterial activity)

Preparations of recommended treatment doses of medications according to the literature and this invention, respectively, are applied to 5mm diameter filter paper disks. These disks are then placed on to agar plates freshly inoculated with bacterial cultures of (a) Staphylococcus aureus and (b) Streptococcus mutans. After incubation for 2 days the zones of inhibition of bacterial growth around the disks are measured. The following drugs were tested: (I) Atridox®; (II) Doxycycline solution (DPF/BNF) (25mg/ml); (III) Gentian violet 0,5%; (IV) Hexadent^{®}; (V) Kank-A®; (VI) Formulation A (as described in example 2) 1,5mg/g doxycycline; (VII) control gel of formulation A (example 2) and (VIII) Periostat^{®}, (IX) Tetracycline 10µg commercial disk.

The control gel was the only preparation which had no effect on the bacteria and very little effect was apparent from Kank-A^{®}. Hexadent^{®} and Gentian violet 0,5% showed an inhibition zone about the average for the materials tested. Atridox^{®}, Periostat^{®}, tetracycline disk, Formulation A (as described in example 2) 1,5mg/g doxycycline and Doxycycline solution (DPF/BNF) (25mg/ml) have marked effect on the growth of the bacteria. Formulation A 1,5mg/g doxycycline does show an inhibition in this concentration but no more than Atridox or Periostat (Table 1). It is predictable that inhibition will occur given the nature of the active substance, this is a positive action in the core of ulceration and as shown in example 5 it does not have this marked effect on the bacteria when in the mixed normal flora nor following diffusion from the base of the ulcer or other lesion.

**Table 1: Bacterial activity of commercially available formulations on Staphylococcus aureus and Streptococcus mutans (Diameter of disk was 5mm.).**

| **Products** | **Diameter of circle around disk (mm)** | |
|---|---|---|
| | *S. aureus* | *S. mutans* |
| **Atridox^{®}** | 18 | 23,5 |
| **Doxycycline solution** **(DPF/BNF) (25mg/ml)** | 26 | 32 |
| **Gentian violet 0.5%** | 12 | 13 |
| **Hexadent^{®}** | 6,5 | 11 |
| **Kank-A^{®}** | 2 | 2 |
| **Formulation A (example 2) 1,5mg/g doxycycline** | 14 | 29 |
| **Control gel of formulation A (example 2)** | 0 | 0 |
| **Periostat^{®} 2mg/ml** | 19 | 26 |
| **Tetracycline disk 10µg** | 18 | 28 |

### Example 4 (Bacterial-activity)

To check the activity of the gel on the growth of oral bacteria two tests were preformed; (i) standard tube dilation test to determine the minimum inhibitory concentration of gel and (ii) a test of the sensitivity of oral microorganisms, in mixed culture to disks impregnated with the gel.
(i) The test organisms for the tube dilation study were *Staphylococcus aureus, Streptococcus sanguis* and *Streptococcus mutans* and 1 g of each gel formulation was diluted in Todd-Hewitt broth in a series of 5 doubling dilutions. Each tube was inoculated with 25 µl of the test organism. The MIC was recorded as the highest dilution that inhibited visible growth in the test tube. Subculture from the tube on to blood agar plates was preformed to determine the minimum bactericidal concentration. None of the formulations other than the one containing chlorhexidine had any observable inhibitory effect on the test organism whereas the formulation containing chlorhexidine inhibited the test strains at all concentrations.
(ii) Disks impregnated with each formulation were placed onto blood agar plates freshly inoculated with mixed oral bacteria collected from healthy volunteers by chewing wax and spitting out 1 ml. of saliva. Incubation of the agar plates was for 48 hours in candle jars. After incubation the plates were examined for zones around the disks that would suggest that the mixed flora or a component of it had been inhibited by the formulation. No inhibition was seen except for the formulation containing chlorhexidine.

### Example 5 (Effect on the normal flora)

Samples were taken from following mucosal areas: (I) buccal mucosa; (II) nasal mucosa; (III) eye mucosa. The test is done according to (ii) described in example 4. The following commercially available preparations were tested on normal flora from (I) buccal mucosa; (II) nasal mucosa; (III) eye mucosa; Atridox^{®}, Bonjela^{®}, Dexocort^{®}, Doxycycline solution (DPF/BNF) (25mg/ml), Gentian violet 0,5%, Hexadent^{®}, Kank-A^{®}, Kenalog^{®}, Listerine^{®}, Formulation A (example 2) 1,5mg/g doxycycline, control gel of formulation A (example 2) and Periostat^{®}.

### Buccal flora:

Bonjela^{®}, Dexocort^{®}, Kank-A^{®}, Kenalog^{®}, control gel (example 2) and Listerine^{®} did not show any inhibiting effect. Doxycycline solution (DPF/BNF) (25mg/ml) and Atridox^{®} showed much inhibiting effect, but Hexadent^{®}, Gentian violet 0,5% and Periostat^{®} had average inhibiting effect compared with the other preparations [Table 2]. Formulation A (example 2) 1,5mg/g doxycycline showed no inhibiting effect apart from a faint circle around the disk which might have been an inhibition of some strains of streptococci. (B1-B5 represents samples from 5 individuals)

**Table 2: Effect on buccal flora. 1) Inner / outer circle**

| **Products** | **Diameter of circle around disk (mm)** | | | | |
|---|---|---|---|---|---|
| | **B1** | **B2** | **B3** | **B4** | **B5** |
| **Atridox^{®}** | 15,5 | 23 | 6 | 11 | 9,5 |
| **Bonjela^{®}** | 0 | 0 | 0 | 0 | 0 |
| **Dexocort^{®}** | 0 | 0 | 0 | 0 | 0 |
| **Formulation A (example 2) 1,5mg/g doxycycline** | 0/6,5¹ | 0/7 | 0/5 | 0/8,5 | 0/0 |
| **Doxycycline solution (DPF/BNF) (25mg/ml)** | 12 | 6 | 14 | 14,5 | 4,5 |
| **Gentian violet 0.5%** | 5,5 | 4 | 6 | 5 | 5,5 |
| **Hexadent^{®}** | 3,5 | 3 | 3,5 | 3 | 3 |
| **Rank-A^{®}** | 0 | 0 | 0 | 0 | 0 |
| **Kenalog^{®}** | 0 | 0 | 0 | 0 | 0 |
| **Control gel of formulation A (example 2)** | 0 | 0 | 0 | 0 | 0 |
| **Listerine^{®}** | 0 | 0 | 0 | 0 | 0 |
| **Periostat^{®} 2mg/ml** | 6/20 | 4/16 | 4/12,5 | 3,5/13,5 | 0/0 |

### Nasal flora:

Bonjela®, Dexocort® and Kenalog® did not have any inhibiting effect but Listerine® and control gel (example 2) did have a minor inhibiting effect in one sample. Kank-A® and Hexadent® had some inhibiting effect. Atridox®, Doxycycline solution (DPF/BNF) (25mg/ml), Periostat® and Gentian violet 0,5% had great inhibiting effect.

Formulation A (example 2) 1,5mg/g doxycycline showed about half the inhibiting effect of the other preparations [Table 3].

**Table 3: Effect on nasal flora.**

| **Products** | **Diameter of circle around disk (mm)** | | | | |
|---|---|---|---|---|---|
| | **N1** | **N2** | **N3** | **N4** | **N5** |
| **Atridox^{®}** | 28 | 23 | 29,5 | 25 | 27 |
| **Bonjela^{®}** | 0 | 0 | 0 | 0 | 0 |
| **Dexocort^{®}** | 0 | 3 | 0 | 0 | 0 |
| **Formulation A (example 2) 1,5mg/g doxycycline** | 8 | 12,2 | 16 | 22,5 | 14 |
| **Doxycycline solution (DPF/BNF) (25mg/ml)** | 33,6 | 26 | 19,6 | 25,6 | 30 |
| **Gentian violet 0.5%** | 16,5 | 14,5 | 23 | 15 | 17,5 |
| **Hexadent^{®}** | 9 | 8 | 9 | 10,3 | 8,5 |
| **Kank-A^{®}** | 7 | 3 | 4,5 | 4,5 | 7 |
| **Kenalog^{®}** | 0 | 0 | 0 | 0 | 0 |
| **Control gel of formulation A (example 2)** | 0 | 0 | 0 | 4,5 | 0 |
| **Listerine^{®}** | 0 | 0 | 0 | 0 | 2,5 |
| **Periostat^{®} 2mg/ml** | 24,5 | 26 | 41 | 30 | 34,3 |

### Eye flora:

No natural flora was cultured from the samples from the eye mucosa. The reason for this is probably the effect of lysozyme that is present in the eye and has bactericidal effect to protect the eye from bacteria.

### Example 6 (Bioadhesion)

In measuring the bioadhesion of the formulations the TA.XT2 Texture Analyser from Stable Micro Systems (England) was used. This apparatus measures and calculates the detachment force needed to pull the composition from a membrane.
The membranes used for bioadhesive measurements were synthetic molecular porous membrane (Spectra/Por^{®} Membrane MWCO: 12-14,000) and pig buccal mucous membrane as well as intestinal membrane.
Bioadhesion has been evaluated for the ingredients of the composition alone and in various formulations, and it has been found that carbopol has good bioadhesive properties alone compared to many other compounds (Nielsen et al., *European Journal of Pharmaceutical Science,* 6, 231-239, 1998). The bioadhesive properties of the compositions, where the peak detachment force and area under force time curve were measured with the TA.XT2 Texture Analyser (Stable Micro Systems, England) and then the work of adhesion was calculated. The formulas which are examined are (I) formulation A (example 2) and (II) formulation B (example 2).
An artificial membrane was used as the receiver substrate (Spectra/Por^{®} Membrane MWCO: 12-14,000).

To keep the volume of gels applied constant, they were measured and applied to the probes using a syringe.
The settings of the TA-XT2 were as follows:
Probe lowering speed: 0,1 mm/s
Contact force: 0,2N
Contact time: 30 s
Probe withdrawal rate: 0,1 mm/s
Withdrawal height: 10 mm
Contact area: 201 mm2

Each experiment was carried out three times.
The force needed to detach the hydrogel was recorded as a function of elongation and both maximum strength and area under force/time curve were obtained. The results were converted into work of adhesion (mJ/cm2) and then represented as a mean value with standard deviation (Table 4). Formulations containing xanthan demonstrated the highest work of adhesion (table 1) although the peak detachment force was low; this is due of the nature of the composition. The binding found for formulations containing polyacrylic acid is markedly greater than what Nielsen (Nielsen et al., European Journal of Pharmaceutical Science, 6, 231-239, 1998) found with Carbopol 934 alone (0,037 ±0,020) or HPMC alone (0,036±0,013) as polymer film at the end of a probe using pig intestine as a receiver membrane.
The results, obtained using an artificial membrane, indicate that this method of measuring hydrogel adhesion gives reliable results.

**Table 4. Work of adhesion calculated from the data produced by TA-XT2.**

| **COMPOSITION** | **Peak detachment force (N)** | **Area under force/time curve (Ns)** | **Work of adhesion (mJ/cm2)** | **SD** |
|---|---|---|---|---|
| **Formulation B** | 0,178 | 1,446 | 0,073 | 0,0012 |
| **Formulation A** | 0,375 | 1,322 | 0,066 | 0,0159 |

### Example 7 (Drug release)

A release study was preformed using disk assembly described in paddle over disk method in USP / NF XXIII, adjusted to a 100ml. Receptor phase was a phosphate buffer pH6,8 and the system was maintained at 37°C. Samples were taken from the system at times; 0,5 , 1, 5, 10, 15, 30, 45, 60, 120, 180, 240, 300 minutes. T Samples were analyzed using a HPLC method.

### Results:

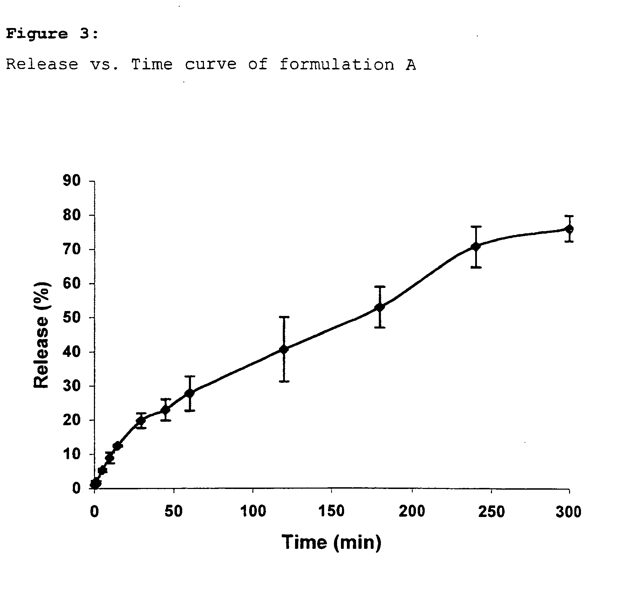

Release figures are in table 6 and a release profile is in figure 3.

**Table 6: Results of the drug release study of formulation A.**

| **Time (min)** | **Release I (%)** | **Release II (%)** | **Release III (%)** | **Mean Release (%)** | **SD** |
|---|---|---|---|---|---|
| **0,5** | 0,977836702 | 0,937251661 | 1,041079872 | 0,985389412 | 0,052324534 |
| **1** | 2, 090403502 | 1,952867211 | 1,377955373 | 1,807075362 | 0,377937867 |
| **5** | 4,769138448 | 5,346195566 | 5,810902704 | 5,308745573 | 0,521890858 |
| **10** | 7,406709429 | 10, 6352697 | 8,951316144 | 8,940517514 | 1,528437381 |
| **15** | 12,18791336 | 12,73224295 | 12,47775093 | 12,46596908 | 0,272355986 |
| **30** | 22,16514284 | 17,70554743 | 19,71048752 | 19,8603926 | 2,233573696 |
| **45** | 20,19828179 | 22,61826758 | 26,23893128 | 23,01849355 | 3,040147559 |
| **60** | 22,3038085 | 28,92396024 | 32,11835113 | 27,78203995 | 5,005926307 |
| **120** | 37,00453446 | 33,62068026 | 51,42546128 | 40,68355866 | 9,955367318 |
| **180** | 58,49448212 | 46,54663203 | 54,25473829 | 53,09861748 | 6,05724706 |
| **240** | 74,44968231 | 64,08570217 | 74,58967925 | 71,04168791 | 6,02446703 |
| **300** | 80,75272063 | 74,44177708 | 74,09968793 | 76,43139521 | 3,746284326 |

### Example 8 (Drug diffusion)

Applying formulation A according to the invention to a synthetic dialysis membrane (Spectra/Por® Membrane MWCO: 12-14,000), drug diffusion is evaluated by using cells based on Franz design. Pieces of membrane were mounted in vertical diffusion cells, based on Franz design. The receptor medium consisted of isotonic phosphate buffer pH 7,4. Receptor solutions were maintained at 37°C and constantly stirred with a magnetic bar. Gel was applied to the membrane and samples withdrawn periodically over 4 h, and refilled immediately with fresh buffer, a final sample was taken at 24 h. Samples were analyzed using a HPLC method.

### Results:

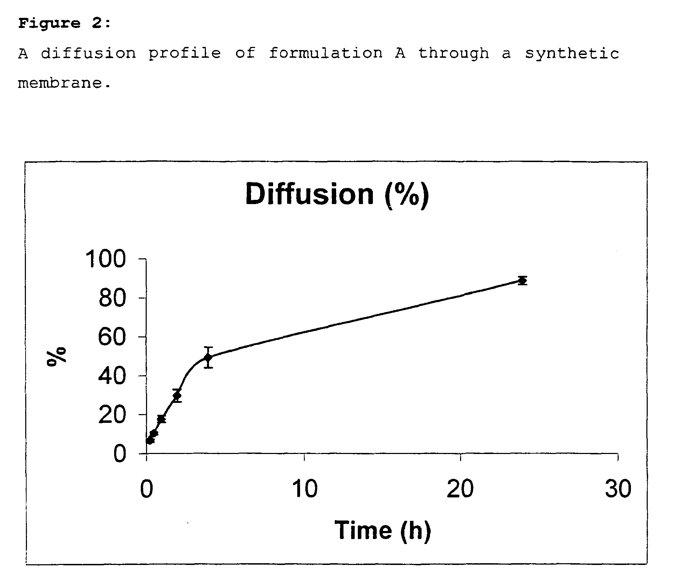

Diffusion figures are in table 5 and profile in figure 2.

**Table 5: Results of diffusion analysis through a synthetic membrane**

| **Time (h)** | **Diffusion I** | **Diffusion II** | **Diffusion III** | **Mean diffusion (%)** | **SD** |
|---|---|---|---|---|---|
| **0,25** | 7,228063 | 6,297016 | 5,983981 | 6,503019923 | 0,647119 |
| **0,5** | 10,86487 | 9,930168 | 9,461612 | 10,0855511 | 0,714418 |
| **1** | 19,12093 | 17,33472 | 15,81758 | 17,42441027 | 1,653497 |
| **2** | 32,80432 | 29,59564 | 26,33029 | 29,57675166 | 3,237056 |
| **4** | 53,59996 | 50,68104 | 43,2434 | 49,17479874 | 5,340055 |
| **24** | 90,51459 | 88,7762 | 86,49847 | 88,59642035 | 2,014086 |

## Claims

1. A bioadhesive pharmaceutical hydrogel composition for treatment of mucosal epithelial ulceration and/or erosions; said composition comprising:
(i) a bioadhesive substance;
(ii) a biologically active substance having MMP inhibitory activity in the range 0.05 to 0.5% (w/w) of the composition; and
(iii) optionally physiologically acceptable vehicle;
said bioadhesive substance and optional physiologically acceptable vehicle being selected to release the biologically active substance in a therapeutic amount into the epithelia of the mucosa without affecting the normal flora thereof.

2. The composition according to claim 1 wherein the biologically active substance is released in a release profile comprising a release of about 10% within at least 10 min.

3. The composition according to claim 1 or 2 wherein the biologically active substance is released in a release profile comprising a release of about 20% within at least 30 min.

4. The composition according to any of claims 1-3 wherein the concentration of the biologically active substance is in the range 0.05 to 0.5%, preferably 0.1 to 0.5%, most preferred 0.1 to 0.3% (w/w) of the composition.

5. The composition according to any of claims 1-4 wherein the concentration of the bioadhesive substance is in the range 0.1 to 8%, preferably 0.1 to 5% (w/w) of the composition.

6. The composition according to any one of claims 1-5 wherein the hydrogel composition has bioadhesive properties to mucosal membranes.

7. The composition according to any of claims 1-6 further comprising one or more bioadhesive components selected from the group consisting of: polysaccharides, such as hydroxymethylcellulose, hydroxypropylmethylcellulose, hydroxyethylcellulose, carboxymethylcellulose, and salts thereof; acrylic polymers such as polyacrylic acids, polymethacrylates, poly(hydroxyethyl methacrylate), poly(methoxyethyl methacrylate), poly(methoxyethoxyethyl methacrylate); proteins such as gelatin; high molecular weight polyhydroxy compounds such as polyvinyl alcohols; high average molecular weight polyalkylene glycols such as polyethylene glycols, optionally cross-linked in particular being with an average molecular weight from about 20,000 to about 4,000,000, propylene glycole, optionally cross-linked with hydrophilic or hydrophobic groups such as glycofurolum; polyvinylpyrrolidones, in particular having an average molecular weight in the range from 10,000 to about 700,000; chitosans, pectins, xanthan gums, carragenans, locust bean gums, acacia gums, and alginates.

8. The composition according to any of claims 1-7 wherein the bioadhesive substance comprises one or more bioadhesive components selected from the group of bioadhesives/water gelling agents such as Carbopol 934, Carbopol 940, Carbopol 941, Carbopol 971, Carbopol 974, Carbopol 980, Carbopol 981, Carbopol 1342, Carbopol 1382, carboxymethylcellulose and hydroxypropylmethylcellulose and salts thereof, xanthan gums, polycarbophil and mixtures thereof.

9. The composition according to any of claims 1-8 wherein the biologically active substance is in a dissolved form.

10. The composition according to any of claims 1-9 wherein the biologically active substance is in a particulate form.

11. The composition according to any of claims 1-10 wherein the biologically active substance is selected from the group consisting of tetracyclines or its derivatives such as tetracycline, doxycycline, minocycline or oxytetracycline, and in a concentration below antimicrobial concentration.

12. The composition according to any of claims 1-11 further comprising one or more additional active ingredients.

13. The composition according to claim 12 wherein the one or more additional active ingredients is selected from the group consisting of anesthetics including benzocaine, pramoxine, dibucaine, diclonine, lidocaine, mepiracaine, prilocaine and tetracaine; astringents including calamine, zinc oxide, tannic acid, hamamelis water, zinc sulfate; wound cleansers such as benzalkonium chloride, carbamide peroxide, tannic acid, salicylic acid, triclosan, benzoyl peroxide and boric acid; wound healing agents such as fish oils, shark liver oil, castor oil, sucralfate and liver yeast cell derivatives; antihistamines such as diphenhydramine, promethazine, cromolyn, cyproheptadine, and azatadine.

14. The composition according to any of claims 1-13 further comprising one or more additional ingredients.

15. The composition according to claim 14 wherein the one or more additional ingredients is selected from the group consisting of surfactants, water absorbing polymers, substances which inhibit enzymatic degradation; organic solvents such as alcohols, oils, pH-controlling agents, solubilizers, stabilizers, HLB-controlling agents, viscosity controlling agents, preservatives, osmotic pressure controlling agents, propellants, air displacement, water, and mixtures thereof.

16. Use of a composition according to Claim 1-15 for the manufacture of a medicament for treatment of mucosal epithelial ulceration and/or erosion in humans.

17. Use according to claim 16 wherein the administration is to a surface of a mucosa.

18. Use according to claim 17 wherein the mucosal surface include parts of the mucosa that is inflamed, ulcerated or surface eroded, said ulcerated and surface eroded mucosa wholly or partly extending through the surface epithelial layer of the mucosa but only extending just into the underlying lamina propria.

19. Use according to claim 16-18 wherein the mucosal surface is selected from the group of mucosal surfaces of the nose, lungs, mouth, eye, ear, gastrointestinal tract, genital tract, vagina and rectum.

## Patentansprüche

1. Bioadhäsive pharmazeutische Hydrogel-Zusammensetzung für die Behandlung von Schleimhautepithel-Ulzeration und/oder -Erosionen; welche Zusammensetzung umfasst:
(i) eine bioadhäsive Substanz;
(ii) eine biologisch aktive Substanz mit MMP-inhibitorischer Aktivität im Bereich von 0,05 bis 0,5 % (w/w) der Zusammensetzung; und
(iii) wahlweise ein physiologisch akzeptables Vehikel;
wobei die bioadhäsive Substanz und wahlweise physiologisch akzeptable Vehikel daraufhin ausgewählt ist, die biologisch aktive Substanz in einer therapeutischen Menge in die Epithelien der Schleimhaut ohne Beeinträchtigung ihrer normalen Flora freizusetzen.

2. Zusammensetzung nach Anspruch 1, wobei die biologisch aktive Substanz bei einem Freisetzungsprofil freigesetzt wird, umfassend eine Freisetzung von etwa 10 % innerhalb von mindestens 10 Min.

3. Zusammensetzung nach Anspruch 1 oder 2, wobei die biologisch aktive Substanz bei einem Freisetzungsprofil freigesetzt wird, umfassend eine Freisetzung von etwa 20 % innerhalb von mindestens 30 Min.

4. Zusammensetzung nach einem der Ansprüche 1-3, worin die Konzentration der biologisch aktiven Substanz im Bereich von 0,05 bis 0,5 %, vorzugsweise 0,1 bis 0,5 %, am bevorzugtesten 0,1 bis 0,3 % (w/w) der Zusammensetzung liegt.

5. Zusammensetzung nach einem der Ansprüche 1-4, worin die Konzentration der bioadhäsiven Substanz im Bereich von 0,1 bis 8 %, vorzugsweise 0,1 bis 5 % (w/w) der Zusammensetzung liegt.

6. Zusammensetzung nach einem der Ansprüche 1-5, worin die Hydrogel-Zusammensetzung bioadhäsive Eigenschaften an Schleimhautmembranen aufweist.

7. Zusammensetzung nach einem der Ansprüche 1-6, außerdem umfassend ein oder mehrere bioadhäsive Komponenten, ausgewählt aus der Gruppe, bestehend aus: Polysacchariden, wie Hydroxymethylcellulose, Hydroxypropylmethylcellulose, Hydroxyethylcellulose, Carboxymethylcellulose, und Salze davon; Acrylpolymere, wie Polyacrylsäuren, Polymethacrylate, Poly(hydroxyethylmethacrylat), Poly(methoxyethylmethacrylat), Poly(methoxyethoxyethylmethacrylat); Proteine, wie Gelatine; hochmolekulare Polyhydroxy-Verbindungen, wie Polyvinylalkohole; Polyalkylenglycole mit hohem mittleren Molekulargewicht, wie Polyethylenglycole, wahlweise quervernetzt, insbesondere mit einem mittleren Molekulargewicht von etwa 20.000 bis etwa 4.000.000, Propylenglycol, wahlweise quervernetzt mit hydrophilen oder hydrophoben Gruppen, wie Glycofurolum; Polyvinylpyrrolidonen, insbesondere mit einem mittleren Molekulargewicht im Bereich von 10.000 bis etwa 700.000; Chitosanen, Pectinen, Xanthangummis, Carragenanen, Johannisbrotkernmehl, Akaziengummis und Alginaten.

8. Zusammensetzung nach einem der Ansprüche 1-7, worin die bioadhäsive Substanz ein oder mehrere bioadhäsive Komponenten, ausgewählt aus der Gruppe der Bioadhäsive/Wasser-Gelbildner, wie Carbopol 934, Carbopol 940, Carbopol 941, Carbopol 971, Carbopol 974, Carbopol 980, Carbopol 981, Carbopol 1342, Carbopol 1382, Carboxymethylcellulose und Hydroxypropylmethylcellulose und Salze davon, Xanthangummis, Polycarbophil und Gemische davon umfasst.

9. Zusammensetzung nach einem der Ansprüche 1-8, worin die biologisch aktive Substanz in einer gelösten Form vorliegt.

10. Zusammensetzung nach einem der Ansprüche 1-9, worin die biologisch aktive Substanz in einer partikulären Form vorliegt.

11. Zusammensetzung nach einem der Ansprüche 1-10, worin die biologisch aktive Substanz ausgewählt ist aus der Gruppe, bestehend aus Tetracyclinen oder deren Derivaten, wie etwa Tetracyclin, Doxycyclin, Minocyclin oder Oxytetracyclin, und in einer Konzentration unterhalb der antimikrobiellen Konzentration.

12. Zusammensetzung nach einem der Ansprüche 1-11, außerdem umfassend ein oder mehrere zusätzliche aktive Inhaltsstoffe.

13. Zusammensetzung nach Anspruch 12, worin die ein oder mehreren zusätzlichen aktiven Inhaltsstoffe ausgewählt sind aus der Gruppe, bestehend aus Anästhetika, einschließlich Benzocain, Pramoxin, Dibucain, Diclonin, Lidocain, Mepiracain, Prilocain und Tetracain; Adstringentien, einschließlich Calamin, Zinkoxid, Tanninsäure, Hamameliswasser, Zinksulfat; Wundreinigern, wie Benzalkoniumchlorid, Carbamidperoxid, Tanninsäure, Salicylsäure, Triclosan, Benzoylperoxid und Borsäure, Wundheilmitteln, wie Fischölen, Haileberöl, Castoröl, Sucralfat und Leberhefezell-Derivaten; Antihistaminen, wie Diphenhydramin, Promethazin, Cromolyn, Cyproheptadin und Azatadin.

14. Zusammensetzung nach einem der Ansprüche 1-13, außerdem umfassend ein oder mehrere zusätzliche Inhaltsstoffe.

15. Zusammensetzung nach Anspruch 14, worin die ein oder mehreren zusätzlichen Inhaltsstoffe ausgewählt sind aus der Gruppe, bestehend aus grenzflächenaktiven Mitteln, wasserabsorbierenden Polymeren, Substanzen, die den enzymatischen Abbau hemmen; organischen Lösungsmitteln, wie Alkoholen, Ölen, pHkontrollierenden Mitteln, Löslichmachern, Stabilisatoren, HLB-kontrollierenden Mitteln, Viskositätskontrollmitteln, Konservierungsstoffen, osmotischen Druckkontrollierenden Mitteln, Treibmitteln, luftverdrängenden Mitteln, Wasser und Gemischen davon.

16. Verwendung einer Zusammensetzung nach Anspruch 1-15 für die Herstellung eines Medikaments zur Behandlung von Schleimhautepithel-Ulzeration und/oder -Erosion beim Menschen.

17. Verwendung nach Anspruch 16, wobei die Verabreichung auf eine Oberfläche einer Schleimhaut ist.

18. Verwendung nach Anspruch 17, wobei die Schleimhautoberfläche Teile der Schleimhaut umfasst, die entzündet, ulzeriert oder oberflächenerodiert ist, welche ulzerierte oder oberflächenerodierte Schleimhaut sich vollständig oder teilweise durch die Oberflächenepithelschicht der Schleimhaut erstreckt, jedoch lediglich bis gerade in die darunter liegende Lamina propria reicht.

19. Verwendung nach Anspruch 16-18, worin die Schleimhautoberfläche ausgewählt ist aus der Gruppe der Schleimhautoberflächen der Nase, Lungen, Mund, Auge, Ohr, Magen-Darm-Trakt, Genitaltrakt, Vagina und Rektum.

## Revendications

1. Composition d'hydrogel pharmaceutique bio adhésive pour le traitement de l'ulcération et/ou des érosions épithéliales de la muqueuse; ladite composition comprenant :
i) une substance bio adhésive;
ii) une substance biologiquement active ayant une activité inhibitrice de MMP dans la gamme de 0,05 à 0,5% (m/m) de la composition; et
iii) éventuellement un véhicule physiologiquement acceptable;
ladite substance bio adhésive et ledit véhicule physiologiquement acceptable éventuel étant choisis pour libérer la substance biologiquement active en quantité thérapeutique dans l'épithélium de la muqueuse sans affecter la flore normale de celle-ci.

2. Composition selon la revendication 1 dans laquelle la substance biologiquement active est libérée avec un profil de libération comprenant une libération d'environ 10% en au moins 10 min.

3. Composition selon la revendication 1 ou 2 dans laquelle la substance biologiquement active est libérée avec un profil de libération comprenant une libération d'environ 20% en au moins 30 min.

4. Composition selon l'une des revendications 1-3 dans laquelle la concentration de la substance biologiquement active est dans la gamme de 0,05 à 0,5%, préférentiellement de 0,1 à 0,5%, plus préférentiellement de 0,1 à 0,3% (m/m) de la composition.

5. Composition selon l'une des revendications 1-4 dans laquelle la concentration de la substance bio adhésive est dans la gamme de 0,1 à 8%, préférentiellement de 0,1 à 5% (m/m) de la composition.

6. Composition selon l'une des revendications 1-5 dans laquelle la composition d'hydrogel a des propriétés bio adhésives aux membranes de la muqueuse.

7. Composition selon l'une des revendications 1-6 comprenant un composant bio adhésif ou plus choisi parmi le groupe comprenant: des polysaccharides tels que de l'hydroxyméthylcellulose, de l'hydroxypropylméthylcellulose, de l'hydroxyéthylcellulose, de la carboxyméthylcellulose, et des sels de ceux-ci; des polymères acryliques tels que des acides polyacryliques, des polyméthacrylates, du poly(hydroxyéthyl méthacrylate), du poly(méthoxyéthyl méthacrylate), du poly(méthoxyéthoxyéthyl méthacrylate; des protéines telles que de la gélatine; des composés polyhydroxy de haut poids moléculaire tels que des alcools polyvinyliques; des polyalkènes glycols de haut poids moléculaire moyen tels que des polyéthylène glycols, éventuellement réticulés en particulier ayant un poids moléculaire moyen d'environ 20 000 à 4 000 000, du polypropylène glycol, éventuellement réticulé avec des groupes hydrophiles ou hydrophobes tels que du glycofurol; des polyvinylpyrrolidones, en particulier ayant un poids moléculaire moyen dans la gamme de 10 000 à environ 700 000; des chitosanes, des pectines, des gommes xanthanes, des carraghénines, des gommes de caroube, des gomme d'acacia et des alginates.

8. Composition selon l'une des revendications 1 à 7 dans laquelle la substance bio adhésive comprend un composant bio adhésif ou plus choisi parmi le groupe des agents gélifiants bio adhésifs/eau tels que du Carbopol 934, du Carbopol 940, du Carbopol 941, du Carbopol 971, du Carbopol 974, du Carbopol 980, du Carbopol 981, du Carbopol 1342, du Carbopol 1382, de la carboxyméthylcellulose et de l'hydroxypropylméthylcellulose et des sels de ceux-ci, des gommes xanthanes, du polycarbophyle et des mélanges de ceux-ci.

9. Composition selon l'une des revendications 1-8 dans laquelle la substance biologiquement active est sous une forme dissoute.

10. Composition selon l'une des revendications 1-9 dans laquelle la substance biologiquement active est sous une forme particulaire.

11. Composition selon l'une des revendications 1-10 dans laquelle la substance biologiquement active est choisie parmi le groupe comprenant des tétracyclines ou ses dérivés tels que de la tétracycline, de la doxycycline, de la minocycline ou de l'oxytétracycline et en concentration inférieure à la concentration antimicrobienne.

12. Composition selon l'une des revendications 1-11 comprenant en plus un ingrédient actif additionnel ou plus.

13. Composition selon la revendication 12 dans laquelle l'ingrédient actif additionnel ou plus est choisi parmi le groupe comprenant des anesthésiques incluant de la benzocaïne, de la pramoxine, de la dibucaïne, de la diclonine, de la lidocaïne, de la mépiracaïne, de la prilocaïne et de la tétracaïne; des astringents incluant de la calamine, de l'oxyde de zinc, de l'acide tannique, de l'eau d'hamamélis, du sulfate de zinc; des nettoyants de plaie tels que du chlorure de benzalkonium, du peroxyde de carbamide, de l'acide tannique, de l'acide salicylique, du triclosan, du peroxyde de benzoyle et de l'acide borique; des agents cicatrisants tels que des huiles de poisson, de l'huile de foie de requin, de l'huile de ricin, du sucralfate et des dérivés de cellule de levure de foie; des antihistamines tels que de la diphenhydramine, de la prométhazine, de la cromolyne, de la cyproheptadine et de l'azatadine.

14. Composition selon l'une des revendications 1-13 comprenant en plus un ingrédient additionnel ou plus.

15. Composition selon la revendication 14 dans laquelle l'ingrédient additionnel ou plus est choisi parmi le groupe comprenant des surfactants, des polymères absorbant l'eau, des substances qui inhibent la dégradation enzymatique; des solvants organiques tels que des alcools, des huiles, des agents de contrôle du pH, des solubilisants, des stabilisants, des agents de contrôle de HLB, des agents de contrôle de la viscosité, des conservateurs, des agents de contrôle de la pression osmotique, des propulseurs, des agents de déplacement de l'air, de l'eau et des mélanges de ceux-ci.

16. Utilisation d'une composition selon les revendications 1-15 pour la fabrication d'un médicament pour le traitement de l'ulcération et/ou de l'érosion épithéliale de la muqueuse chez l'homme.

17. Utilisation selon la revendication 16 dans laquelle l'administration est à une surface d'une muqueuse.

18. Utilisation selon la revendication 17 dans laquelle la surface de la muqueuse inclut des parties de la muqueuse qui est enflammée, ulcérée ou érodée en surface, ladite muqueuse ulcérée et érodée en surface s'étendant en totalité ou en partie dans la couche épithéliale de la surface de la muqueuse mais s'étendant juste seulement dans la lamina propria sous jacente.

19. Utilisation selon les revendications 16-18 dans laquelle la surface de la muqueuse est choisie parmi le groupe des surfaces de la muqueuse du nez, des poumons, de la bouche, de l'oeil, de l'oreille, du système gastro-intestinal, du système génital, du vagin et du rectum.
